# EUROPÄISCHE PATENTSCHRIFT

(11) **EP 2 874 569 B1**
(45) Veröffentlichungstag und Bekanntmachung des Hinweises auf die Patenterteilung: **08.06.2016**
(21) Anmeldenummer: 13741672.3
(22) Anmeldetag: 18.07.2013
(51) Int. Cl.: A61F 2/16

(54) **WEITWINKELOPTIK FÜR OPHTHALMOLOGISCHE IMPLANTATE**
WIDE-ANGLE OPTICAL UNIT FOR OPHTHALMOLOGICAL IMPLANTS
OPTIQUE GRAND ANGLE POUR IMPLANTS OPHTALMOLOGIQUES

(30) Priorität: 23.07.2012 DE 102012106653
(43) Veröffentlichungstag der Anmeldung: 27.05.2015
(73) Patentinhaber: Karlsruher Institut für Technologie, 76131 Karlsruhe (DE)
(72) Erfinder: SIEBER, Ingo, 76133 Karlsruhe (DE); GUTH, Helmut, 76344 Eggenstein-Leopoldshafen (DE); BRETTHAUER, Georg, 76139 Karlsruhe (DE); GEGENBACH, Ulrich, 75196 Remchingen/Singen (DE); GUTHOFF, Rudolf F., 18119 Rostock (DE)
(86) Internationale Anmeldenummer: PCT/EP2013/002139
(87) Internationale Veröffentlichungsnummer: WO 2014/015964

(56) Entgegenhaltungen:
- EP-A1- 2 196 172
- DE-A1- 19 501 444
- US-B1- 6 358 280

## Beschreibung

Die Erfindung betrifft eine Weitwinkeloptik für ophthalmologische Implantate, gemäß dem ersten Patentanspruch.
Die Weitwinkeloptik ist eine Komponente eines vorzugsweise ophtalmologischen Implantats zur Wiederherstellung der Akkommodationsfähigkeit. Sie umfasst eine Optik mit hoher Winkelakzeptanz sowie einer Auskopplungsstruktur. Sie umfasst nicht zwingend den optischen Verstellmechanismus des Akkommodationssystems. Gemeinsam mit dem optischen Verstellmechanismus und ggf. separaten Austrittsflächen oder -komponenten bildet die Weitwinkeloptik ein optisches Systems.
Ophthalmologische Implantate dienen in der Augenheilkunde zur Wiederherstellung und Erhaltung des Sehvermögens. Einfache Implantate sind z.B. künstliche Augenlinsen die z.B. im Falle einer Eintrübung der körpereigenen Augenlinse bei einem Grauen Star durch Austausch dieser ersetzen. Derartige Linsen umfassen eine einzelne Linse. Bekannt sind auch bi- oder multifokale Linsen mit zwei oder mehreren parallel eingestellten festen Brennweiten in der Augenheilkunde, wobei sich der Patient je nach Bedürfnis nach einer Trainingsphase situationsbedingt auf eine der stets angebotenen Brennweiten zugunsten einer höheren Bildschärfe konzentriert. Üblicherweise sind künstliche Augenlinsen einstückig, d.h. sie sind kein Linsensystem mit mehreren Linsen.
Insbesondere aus jüngerer Zeit sind auch verstellbare Linsensysteme bekannt, bei denen entweder in ihrer Form verstellbare Einzellinsen oder verstellbare Linsensysteme zum Einsatz kommen.

Aus der Druckschrift EP-A-2196172 ist eine Weitwinkeloptik für ophthalmologische Implantate bekannt, die die technischen Merkmale des Oberbegriffs aus Anspruch 1, offenbart.
Beispielsweise offenbart die **[1]** ein künstliches System zur Wiederherstellung der Akkommodationsfähigkeit. Dieses umfasst ein unabhängig vom Ziliarmuskel verstellbares optisches System, das entweder ein aktiv optisches Element mit einer veränderbaren Krümmung einer lichtbrechenden Grenzfläche oder einer veränderbaren Brechungindexverteilung oder ein verschiebbares passiv-optisches Element mit unveränderlichen optischen Eigenschaften oder die Kombination eines oder mehrerer aktiver und / oder passiver optischer Elemente ist.

Aus der **[2]** ist ferner ein Augenimplantat mit einer Vielzahl von Linsen in einem im wesentlichen zylinderförmigen Gehäuse bekannt, das über haptische Elemente im Auge eingesetzt wird. Das Implantat zeichnet sich nicht nur durch seinen bevorzugt zylinderförmigen Aufbau zentriert um eine optische Achse, sondern auch durch eine Lichtundurchlässigkeit des Zylindermantels aus.

Komplex aufgebaute optische Systeme wie die vorgenannten, welche an Stelle oder zusätzlich zu der menschlichen Linse im Auge implantiert werden, schränken meist auf Grund ihrer geometrischen und optischen Rahmenbedingungen wie z.B. der durch die Abmessungen im Auge erforderlichen Miniaturisierung das Gesichtsfeld des potentiellen Patienten stark ein. Damit unterscheiden sich die genannten Augenimplantate grundsätzlich von den aus der optischen Messtechnik und/oder aus dem Objektivbereich bekannten Optiken. Das natürliche menschliche Auge weist ein Gesichtsfeld von vertikal ca. 130° und horizontal ca. 180° auf. Dies führt insbesondere dazu, dass bei der Implantation eines komplexen Aufbaus der periphere Visus des Patienten in vertikaler und horizontaler Richtung stark beeinträchtig wird. So ist z.B. bei einem künstlichen Akkommodationssystem, wie es in **[1]** vorgeschlagen wird, mit einem eingeschränkten Gesichtsfeld von maximal 80° zu rechnen, was zu einem sogenannten Tunnelblick führt. Das periphere Sehvermögen ist jedoch für die Orientierung des Menschen im Raum, für die Orientierung in Dämmerung und Dunkelheit sowie für sein Reaktionsvermögen bei seitlichen Reizen unentbehrlich.

Herkömmliche Weitwinkelsysteme und diese enthaltende optische Systeme allein sind für den Einsatz im menschlichen Auge nicht geeignet, da ihr Sinn darin besteht, den Weitwinkelbereich in der Objektebene auf einen definierten, kleinen Bereich in der Bildebene (Film, Kamerachip) abzubilden.

Davon ausgehend liegt die **Aufgabe der Erfindung** darin, eine Weitwinkeloptik für ophthalmologische Implantate im Auge, die aus baulichen bzw. konstruktionsbedingten Gründen das Gesichtsfeld des Patienten einschränken, so zu gestalten, dass sie ohne eine Einschränkung des Sehvermögens in einem zentralen Sichtfeld zugleich auch ein peripheres Sehen des Implantatträgers gewährleisten.

Die Aufgabe wird durch eine Weitwinkeloptik mit den Merkmalen des Anspruch 1 gelöst. Auf diesen rückbezogene Unteransprüche geben vorteilhafte Ausgestaltungen wieder.

Zur Lösung der Aufgabe wird eine Weitwinkeloptik eines optischen Systems für ophthalmologische Implantate im Auge vorgeschlagen, die zumindest ein rotationssymmetrisch um eine optische Achse angeordnetes Linsensystem mit mindestens zwei vorzugsweise flächig aufeinanderliegenden Linsen aus verschiedenen Materialien sowie eine entsprechend der optischen Achse ausgerichtetes System zur Brechkrafteinstellung sowie eine Auskopplungsstruktur aufweist.

Das optische System weist eine distale, d.h. von der Retina weg gerichtete, und eine zur Retina hin gerichtete proximale Seite auf, die jeweils durch eine distale und proximale optische Austrittsfläche gebildet werden. Vorzugsweise ist das Linsensystem der Weitwinkeloptik distalseitig und die Auskoppelungstruktur proximalseitig in dem optischen System angeordnet. Vorzugsweise bildet eine erste Linse des Linsensystems die distale Austrittsfläche und Ankopplungsfläche zu der mit Kammerwasser gefüllten vorderen Augenkammer des Auges. Die proximale Austrittsfläche des optischen Systems grenzt direkt an den Glaskörper an und wird vorzugsweise durch ein Austrittsfenster gebildet. Das Austrittsfenster ist optional als optische Linse, der Nachsatzlinse gestaltet und bildet als solche die proximale Abschlusslinse des Linsensystems zum Glaskörper des Auges hin. Das Austrittsfenster ist vorzugsweise eine integrale Komponente eines Gehäuses des optischen Systems.

Die Brechungen des Lichtstrahls erfolgen an den Grenzflächen zwischen jeweils zwei Abschnitten mit unterschiedlichem Brechungsindex. Eine erste Brechung eines in das Auge einfallenden Lichtstrahls erfolgt folglich an der Außenfläche der gekrümmten Hornhaut des Auges, gefolgt von einer Brechung zwischen Hornhautinnenseite und dem Kammerwasser in der vorderen Augenkammer und anschließend von dort in den Übertritt in die distale Austrittsfläche der distalen Linse des Linsensystems.

Ein wesentliches Merkmal betrifft das Linsensystem insbesondere im Bereich der distalen Austrittsfläche. Es ist so konzipiert, dass ein durch die distale Austrittfläche einfallender Lichtstrahl zur Erfassung auch des peripheren Sichtbereichs auch bei größeren Einfallswinkeln im Bereich zwischen 80-90° oder 80- 95°, bevorzugt zwischen 80-85° durch die Austrittsfläche gebrochen wird und im Linsensystem ohne eine Totalreflexion an den Übertritten zwischen zwei Linsen weitergeleitet wird. Vorzugsweise ist die Austrittsfläche plan gestaltet, wobei der auf das Auge auftreffende Lichtstrahl bei einem Einfallswinkel von 90° parallel zu dieser ausgerichtet ist, jedoch auf Bereiche der natürlichen sphärischen Hornhautoberfläche auftrifft und in diesem Bereich auch gebrochen wird. Erst durch die vorgenannten ersten Lichtbrechungen im Bereich der Hornhaut wird das Linsensystem ertüchtigt, auch bei einer planen Austrittsfläche auch Einfallwinkel von über 80° bis über 90° aufzunehmen.

Die Linsen des Linsensystems weisen hierzu unterschiedliche Brechungsindizes auf, wobei der Brechungsindex je Linse des Linsensystems jeweils zur benachbarten Linse ausgehend von der distalen Austrittsfläche stufenweise verändert, vorzugsweise alterniert.

Die Optik variabler Brechkraft des optischen Systems besteht entweder aus mechanisch verschiebbaren Linsenkombinationen (z.B. Verschiebelinse) und/oder Linsenkörper verstellbarer Brennweite, vorzugsweise in einem mit inertem Gas gefüllten Volumen.

Die Grenzflächen zwischen dem Gas und einem optischen Element (Linse, Austrittsfenster oder sonstige optischen Festkörper) bedeuten jeweils eine signifikante Änderung des Brechungsindex, der für die Einstellung der Brennweite des optischen Systems erforderlich ist. Die Optik hoher Winkelakzeptanz ist durch aufeinander folgende Linsen vorzugsweise alternierender Brechungsindizes so zu gestalten, dass die auf die optischen Grenzflächen auftreffenden Strahlen unterhalb des kritischen Winkels der Totalreflexion liegen.

Wesentliches Merkmal der Weitwinkeloptik ist ferner eine Auskopplungsstruktur, die als separate optische Komponente eine Weiterleitung der Lichtstrahlen insbesondere aus Einfallswinkeln über 40° bis über 90° auf peripheren Bereiche der Netzhaut (Retina) umlenkt.

Eine bevorzugte Ausführungsform weist zudem eine ringförmig umlaufende Grenzfläche auf der das optische System proximal abschließende Linse auf, auf die eine ringförmige optische Auskopplungsstruktur aufgesetzt ist und vorzugsweise optische Güte aufweist. Die Auskopplungsstruktur ist vorzugsweise als Ringprisma gestaltet und als solches vorzugsweise um die proximale Optik variabler Brechkrafteinstellung (z.B. Verschiebelinse) angeordnet. Die Begriffe Auskopplungstruktur und Ringprisma umfassen im Rahmen der Erfindung neben einem geschlossenen Ringelement mit gleichbleibenden oder sich ändernden Struktur- oder Prismenquerschnitt auch Ringsegmente (Teilringprisma), die nur bestimmte Grenzflächenteilabschitte anstelle der gesamten umlaufenden Grenzfläche überdecken. Beispielsweise können auch eine Auskopplungsstruktur, Ringprisma oder mehrere unterschiedliche Ringsegmente mit unterschiedlichen Struktur- oder Prismenquerschnitten auf der Grenzfläche angeordnet sein, die z.B. ein Sichtfeld von 180° in horizontaler Richtung und nur 130° in vertikaler Richtung ermöglichen.

Die Auskopplungsstruktur dient dazu, die Weitwinkelakzeptanz ophthalmologischer Implantate, die aus baulichen bzw. konstruktionsbedingten Gründen das Gesichtsfeld des Patienten einschränken, zu erhöhen und in den Bereich des natürlichen menschlichen Auges zu überführen und somit das periphere Sehen des Implantatträgers zu gewährleisten oder zu erweitern.

Mit der Auskopplungsstruktur wird in vorteilhafter Weise der vom Auge erfassbare Bildbereich in einen zentralen höher aufgelösten Bereich sowie einen niedriger aufgelösten peripheren Randbereich unterteilt und damit an das optische Auflösungsvermögen der Netzhaut angepasst. Das natürliche menschliche Auge besitzt ein Gesichtsfeld (Sichtfeld) von vertikal ca. 130° und horizontal ca. 180° d.h. ca. ±65° vertikal und ca. ±90° horizontal um die Symmetrielinie der Augenlinsen bzw. der Augenausrichtung.

Ein Tunnelblick schränkt das Sichtfeld eines gehäusten ophthalmologischen Implantats ohne weitere Maßnahmen deswegen ein, weil insbesondere Lichtstrahlen aus größeren Feldwinkeln nicht oder nur ungenügend auf die Netzhaut auftreffen.

Insbesondere die Lichtstrahlen, die mit größeren Einfallswinkeln insbesondere über 60° zur Hauptsehrichtung (gleich der Ausrichtung des Auges bzw. Symmetrielinie der Weitwinkeloptik im Auge) in das Auge einstrahlen, werden durch die Optik im Auge entweder nicht weitergeleitet oder innerhalb des vorgenannten eingeschränkten Projektionsbereichs auf der Netzhaut abgebildet.

Mit der Auskopplungsstruktur, insbesondere mit dem genannten Ringprisma lassen sich der Sichtbereich in vorteilhafter Weise in die beiden vorgenannten Teilbereiche aufteilen, was der natürlichen Aufteilung des Sehbereichs in einen zentralen fovealen und einen darum angeordneten peripheren Sehbereich entgegenkommt. Das foveale Sehen liefert einen scharfen Seheindruck eines sehr beschränkten Gesichtsfeldwinkels um die Symmetrielinie der Augenlinsen bzw. der Augenausrichtung. Hierbei wird lediglich ein Sichtbereich von unter 5° (typischerweise 1-2°) auf die Fovea Centralis (Fixationspunkt), dem zentralen Bereich auf der Netzhaut d.h. dem Bereich des schärfsten Sehens beim menschlichen Auge abgebildet. Im Gegensatz zu diesem fovealen Sehen vermittelt das periphere Sehen unscharfe Seheindrücke außerhalb des Fixationspunktes im verbleibenden Winkelbereich des abbildbaren Sehfeldes. Des Weiteren ist die menschliche Retina außerhalb der Fovea Centralis bis hin zu ihren Randbereichen auf Grund ihres Aufbaus sehr effizient bezüglich Objektbewegungen, besitzt eine, im Vergleich zur Fovea Centralis, viel stärkere Hell-Dunkel-Empfindlichkeit und wird daher für das Sehen in Dämmerung und im Dunkeln benötigt. Das periphere Sehen ist also für die Vermittlung des primären Eindrucks der Gesamtheit einer Szenerie verantwortlich, für die Detektion von Objektbewegungen sowie für das Dämmerungssehen.

Beim peripheren Sehen des Menschen müssen die Informationen, welche aus dem Weitwinkelbereich in das Auge eintreffen, auch auf die peripheren Bereiche der Retina abgebildet werden, da nur in den Außenbereichen der Retina die für das periphere Sehen notwendigen Sinneszellen existieren.

Die Form und der Brechungsindexunterschied der Auskopplungsstruktur zum Glaskörper bewirkt eine weitere reflexionsarme Umlenkung des durchtretenden Lichtstrahls vorzugsweise in die peripheren Bereiche der Netzhaut.

Eine Ausführung sieht ein Ringprisma vor, das formschlüssig auf die Grenzfläche aufgesetzt ist und auf dieser vorzugsweise auch fixiert ist. Ist das Ringprisma auf einer proximalseitigen Verschiebelinse angeordnet, bewegt sich diese gemeinsam mit dem Ringprisma gegenüber dem vorzugsweise proximalen gehäuseseitigen Austrittsfenster sowie zum Übergang zum Glaskörper.

Eine alternative Ausführung sieht ein Ringprisma vor, das gegenüber einer proximalseitigen Verschiebelinse (alternativ Linsensystem oder Linse mit variabler Brechkraft) Relativbewegungen zulässt. Das Ringprisma ist nicht schlüssig mit dem Verschiebelinse verbunden, sondern ist vorzugsweise integraler Bestandteil des z.B. gehäusesseitigen Austrittsfensters, umschließt aber vollständig oder teilweise die umlaufende Grenzfläche der proximalen Verschiebelinse. Die Verschiebbarkeit zwischen Verschiebelinse und Ringprisma bewirkt ein sich mit der Verschiebung änderndes und von der Netzhaut erfassbares Sichtfeld.

Die Merkmale der Weitwinkeloptik umfassen folglich für die bestimmte Abmessung des Implantats ausgelegten, verschiedenen refraktiven optischen Linsen unterschiedlicher optischer Materialien und Brechkräfte sowie vorzugsweise einer prismatischen Auskopplungsstruktur, welche die Optik radial umfasst und an die jeweilige Anatomie anpassbar ist. Es liegt im Rahmen der Erfindung, dass in Abhängigkeit z.B. von der patientenspezifischen Anatomie und den Anforderungen an die Optik des Implantats auch nur einzelne Segmente der Auskopplungsstruktur zur Anwendung kommen.

Dem Träger eines komplexen Implantats, das mit der Weitwinkeloptik ausgestattet ist, wird ein peripheres Sehen, wie es durch die natürliche Linse des Menschen gegeben ist, gewährleistet. Dadurch wird der vorgenannte Tunnelblick, verursacht durch die baubedingte Beschränkung des Gesichtsfeldes auf Grund der Implantatsauslegung, behoben und dem Patienten die Orientierung im Raum, die Erfassung der Gesamtszenerie, Reaktionsmöglichkeit auf seitliche Reize sowie das Sehen in Dämmerung und bei wenig Licht ermöglicht. Die Weitwinkeloptik dient der Erhöhung der Sicherheit und der Lebensqualität der Implantatsträger. Wesentliche technische Merkmale sowie Vorteile und Wirkungen der Weitwinkeloptik sind zusammengefasst:
- Kombination mehrerer optischer Komponenten, um das von der Netzhaut erfassbare Sichtfeld zu erhöhen,
- Anordnung der einzelnen optischen Komponenten so, dass auftretende Aberrationen kompensiert werden können,
- prismatische, das optische System mindestens zum Teil umlaufende Strukturen zur Auskopplung und Aufspreizung der Strahlen, welche aus größeren Feldwinkeln in das optische System eindringen, um eine Abbildung in die Peripherie der Retina zu gewährleisten. Die Auskopplungsstruktur ist an die anatomischen Gegebenheiten des Implantatträgers anpassbar.

Die Erfindung wird im Folgenden anhand eines Ausführungsbeispiels in Kombination mit einer axial verschiebbaren Verschiebelinse erläutert, das optional auch mit einzelnen oder allen vorgenannten Maßnahmen zusätzlich kombinierbar oder erweiterbar ist. Es zeigen
**Fig.1** einen Schnitt durch ein Ausführungsbeispiel des optischen Systems mit Weitwinkeloptik und Verschiebelinse sowie
**Fig.2a** **bis r** eine Gegenüberstellung von berechneten Lichtstrahlverläufen an einer herkömmlichen Optik **(a)** bis **(i)** im Vergleich zu einer Weitwinkeloptik **(j)** bis **(r)** in einer Auslegungsform des künstlichen Akkommodationssystems anstelle der natürlichen menschlichen Linse an Hand eines schematischen Modells des menschlichen Auges mit einem optischen System mit einstellbarer Brechkraft.

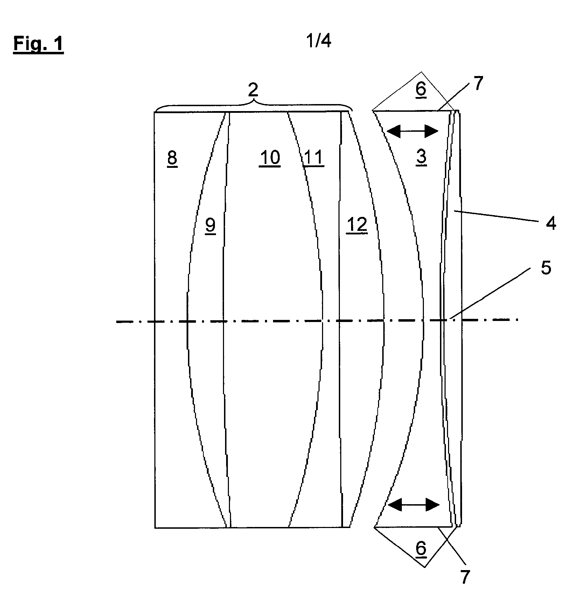

Ein optisches System mit Weitwinkeloptik und Verschiebelinse für ophthalmologische Implantate für das Ausführungsbeispiel eines künstliches Akkommodationssystem zeigt **Fig.1** in einer Schnittdarstellung. Die Brechkraftverstellung des optischen Systems erfolgt in der dargestellen Ausführungsform mit einem dreilinsigen System, umfassend ein Linsensystem **2** mit hoher Winkelakzeptanz, eine Verschiebelinse **3** sowie eine Nachsatzlinse **4** mit einer gemeinsamen optischen Achse **5.** Die im Beispiel bikonkave Verschiebelinse ist entlang der optischen Achse relativ zu dem Linsensystem sowie der Nachsatzlinse verschiebbar. Die Nachsatzlinse gewährleistet eine patientenspezifische Grundbrechkraft des optischen Systems und damit des ophthalmologischen Implantats, erlaubt die Kompensation von Aberrationen und dient als proximales Austrittsfenster für das Akkommodationssystem zum Glaskörper des Auges hin. Das dargestellte optische System umfasst mit dem Linsensystem **2** eine optische Komponente, welche die Winkelakzeptanz des für die Einstellung des gewünschten Akkommodationszustandes notwendigen dreilinsigen optischen Systems erhöht und der Auskopplungsstruktur **6,** welche auf die Grenzfläche **7** um die Verschiebelinse **3** aufgesetzt ist und der Auskopplung und Spreizung der Weitwinkelbereiche zur Netzhaut hin dient.

Das Linsensystem **2** ist mit fünf Einzellinsen **8, 9, 10, 11** und **12,** die flächig aufeinander liegen und vorzugsweise auch gegeneinander fixiert sind, so ausgelegt, dass die Winkelakzeptanz des optischen Gesamtsystems erhöht wird. Hierzu sind die Einzellinsen beispielhaft wie folgt aufgeführt: Die erste Linse **8** ist eine einfachkonkave Linse aus einem Glas mit Brechungsindex 1,716 bei 632,8nm Wellenlänge (N-KZFS8 der Fa. Schott, Mainz, **[3]**), wobei die plane Linsenfläche als distalseitige Austrittsfläche dient. Die zweite Linse **9** ist eine konvex-konkave Linse aus dem farbkorrigierenden Barium-Kronglas mit einem Brechungsindex 1,567 bei 632,8nm Wellenlänge (N-BAK4 der Fa. Schott, Mainz, **[3]**). Im Ausführungsbeispiel liegen die ersten beiden Linsen **8** und **9** mit ihrer Profilierung formschlüsssig aufeinander und bilden zusammengesetzt eine Verbundlinse, an die proximalseitig die dritte Linse **10,** eine bikonvexe Linse formschlüssig anliegt. Die dritte Linse **10** besteht aus einem Glas mit Brechungsindex 1,845 bei 632,8nm Wellenlänge (N-LASF9 der Fa. Schott, Mainz, **[3]**). Gemeinsam mit der dritten bildet eine bikonkave vierte Linse **11** aus einem Glas mit Brechungsindex 1,457 bei 632,8nm Wellenlänge (Lithosil-Q der Fa. Schott, Mainz, **[3]**) ebenfalls eine Verbundlinse, an deren proximaler Linsenfläche formschlüssig die fünfte bikonvexe Linse **12** aus anschließt. Das Linsensystem **2** wird durch die fünfte bikonvexe Linse **12** aus Polymethylmethacrylat mit Brechungsindex 1,508 bei 632,8nm Wellenlänge (PMMA, Acrylic-2 nach **[3]**) proximal zu der Verfahrlinse abgeschlossen.

Dabei setzt sich das Linsensystem zur Erhöhung der Winkelakzeptanz aus Materialien mit unterschiedlichen Brechungsindices und angepassten Krümmungen zusammen um die Totalreflexion an den Materialübergängen zu vermeiden. Im Ausführungsbeispiel werden hierzu alternierende zwischen hohen und niedrigen Brechungsindices gewählt. Die Mindestanzahl der Linsen und Komponenten sowie die Materialwahl sind abhängig von den Mindestanforderungen, welche an das Linsensystem und die Winkelakzeptanz gestellt werden.

Die prismatische Auskopplungsstruktur **6** ist in der dargestellten Ausführung als geschlossene Ringstruktur gewählt. Das Material der Auskopplungsstruktur kann am Material des Gehäuses des oph-thalmologischen Implantats orientiert werden. In dieser Ausführung wurde als Material PMMA gewählt.

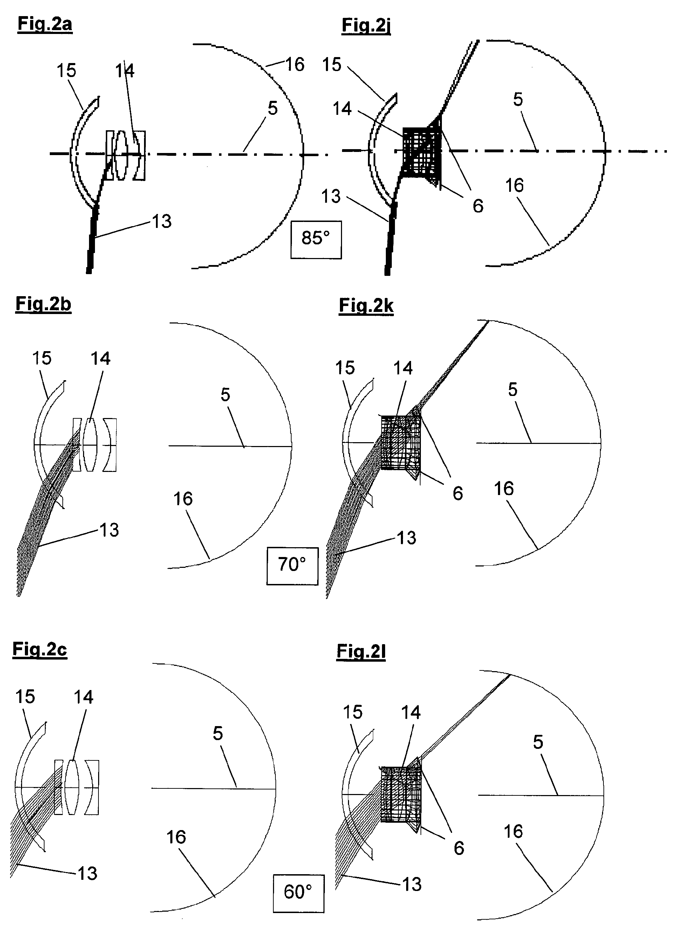

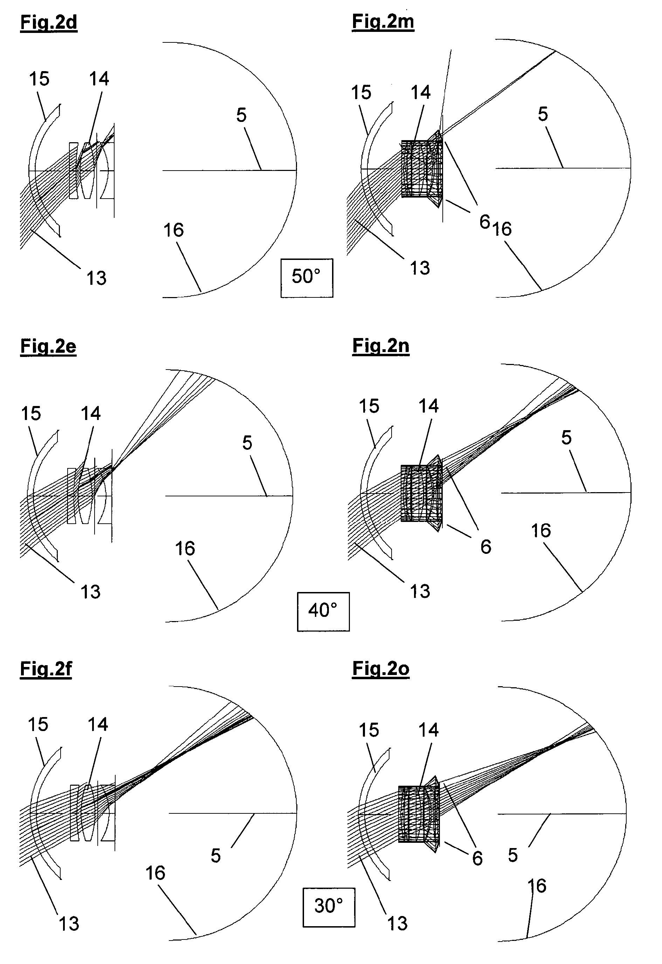

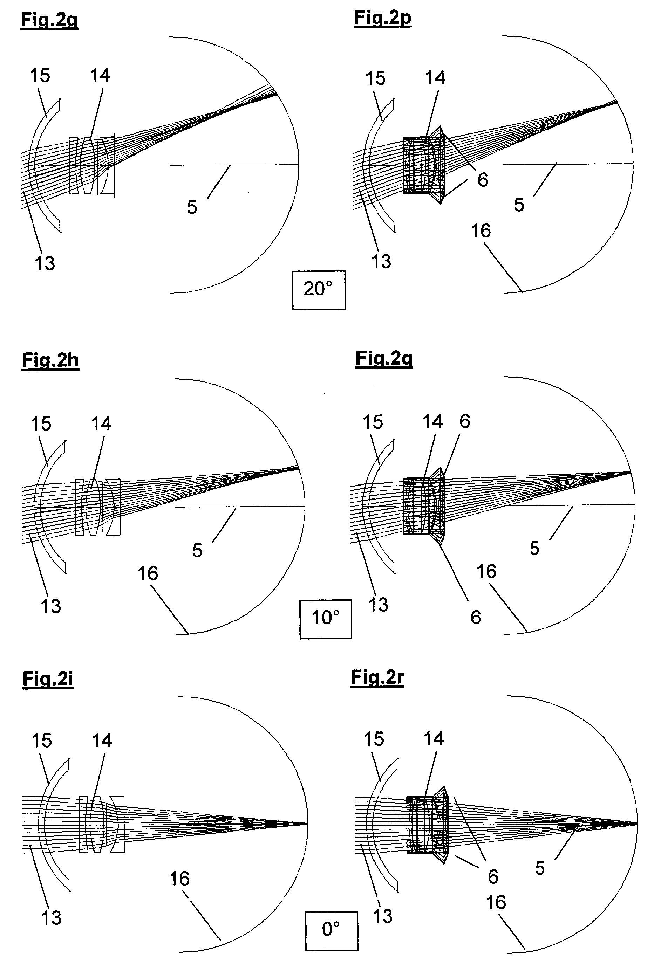

**Fig.2a** **bis r** zeigen eine Gegenüberstellung von berechneten Lichtstrahlverläufen an Hand eines schematischen Modells des menschlichen Auges mit einem optischen System mit einstellbarer Brechkraft in einer Auslegungsform des künstlichen Akkommodationssystems anstelle einer natürlichen menschlichen Linse. Die an Hand eines schematischen Modells des menschlichen Auges. Die Figuren zeigen schematisch den Lichtstrahlverlauf **13** sowie zeilenweise den Einfallswinkel (halber Feldwinkel) und die optische Achse **5** sowie die das optische System **14** im Auge, das durch die Hornhaut **15** sowie die Netzhaut **16** stellvertretend für den Augapfel wiedergegeben ist. Als Optik einstellbarer Brechkraft ist hier eine dreilinsige Optik modelliert. In der linken Spalte **(****Fig.2a** bis **i)** sind die Ergebnisse der Strahlverlaufsberechnungen ohne und in der rechten Spalte **(****Fig.2j** bis **r)** mit der Weitwinkeloptik.

Berechnet sind Strahlen des halben Feldwinkelbereichs 0°-85° in 10° Schritten bis 70° und der Winkel 85°. Der halbe Feldwinkel ist gleich dem Einfallwinkel des auf die Hornhaut **15** einfallenden Lichtstrahls **13** zur optischen Achse **5.** Er ist unter jedem Einzelbildpaar, d.h. zeilenweise in **Fig.2a** bis **r** angegeben. Der Strahlverlauf geht von links nach rechts. Wie in der linken Figurenspalte (ohne Weitwinkeloptik) dargestellt, geht aus berechneten Lichtstrahlverläufen hervor, dass nur Strahlen, die in einem halben Feldwinkel kleiner gleich 40° auf die Hornhaut treffen, überhaupt zur Netzhaut weitergeleitet werden. Strahlen, die unter einem größeren Einfallswinkel auf das optische System treffen, erfahren an den optischen Grenzflächen Totalreflexion, und zwar bei einem Einfallswinkel bei 50° an der Grenzfläche zur der dritten, darüber bereits an der Grenzfläche der ersten Linse. Dies führt zu vorgenanntem Tunnelblick, da Informationen bei Einfallwinkeln größer 40° nicht die Netzhaut erreichen sowie ein insgesamt eingeschränkter Bereich auf der Netzhaut für die Erfassung genutzt wird.

In der rechten Figurenspalte ist das gleiche optische Wirkprinzip für die Brechkraftänderung in Kombination mit der Weitwinkeloptik dargestellt. Die Winkelakzeptanz, d.h. der maximale Einfallswinkel, bis zu dem ein einfallender Lichtstrahl durch die Weitwinkeloptik auf die Netzhaut weitergeleitet wird, wird allein durch die Weitwinkeloptik von 40° auf 85° erhöht. Dabei wird vom Lichtstrahl im Vergleich zur linken Figurenspalte insbesondere auch ein wesentlich größerer Bereich auf der sphärischen Retina (Netzhaut) erfasst, was vom Träger des Augenimplantats subjektiv nicht mehr oder nur in einem deutlich reduzierten Maße als vorgenannten Tunnelblick wahrgenommen wird.

### Literatur

**[1]** WO 2007/020184 A1
**[2]** WO 00/38593 A1
**[3]** www.filmetrics.de/refractive-index-database Brechungsindexdatenbank der Fa. Filmetrics, Inc.

### Bezugszeichenliste

- 2: Linsensystem
- 3: Verschiebelinse
- 4: Nachsatzlinse
- 5: optischen Achse
- 6: Auskopplungsstruktur
- 7: Grenzfläche
- 8: Linse aus N-KZFS8
- 9: Linse aus N-BAK4
- 10: Linse aus N-LASF9
- 11: Linse aus Lithosil-Q
- 12: Linse aus PMMA
- 13: Lichtstrahl
- 14: Optische System
- 15: Hornhaut
- 16: Netzhaut

## Patentansprüche

1. Weitwinkeloptik für ophthalmologische Implantate im Auge, umfassend ein rotationssymmetrisch um eine optische Achse **(5)** angeordnetes Linsensystem **(2),** umfassend mindestens zwei flächig aufeinanderliegende Linsen **(8-12)** aus Materialien unterschiedlicher optischer Brechungsindizes sowie eine Nachsatzlinse**(4)**, die zum Augeninneren proximal angeordnet ist, **dadurch gekennzeichnet,**
**dass** um die Nachsatzlinse (4) eine optische Auskopplungsstruktur **(6)** aufgesetzt ist.

2. Weitwinkeloptik nach Anspruch 1, **dadurch gekennzeichnet, dass** eine zusätzliche mechanisch verschiebbare Linsenkombination **(3)** und/oder Linsenkörper verstellbarer Brennweite vorhanden ist.

3. Weitwinkeloptik nach Anspruch 2, **dadurch gekennzeichnet, dass** die proximal zum Augeninneren angeordnete Nachsatzlinse **(4)** und/oder die verschiebbare Linsenkombination **(3)** und/oder der Linsenkörper verstellbarer Brennweite eine ringförmig umlaufende Grenzfläche aufweist, wobei auf diese die optische Auskopplungsstruktur **(6)** aufgesetzt ist.

4. Weitwinkeloptik nach einem der Ansprüche 1 bis 3, **dadurch gekennzeichnet, dass** die Auskopplungsstruktur **(6)** in Umlaufrichtung einen nicht konstanten Strukturquerschnitt aufweist.

5. Weitwinkeloptik nach einem der Ansprüche 1 bis 4, **dadurch gekennzeichnet, dass** die Auskopplungsstruktur **(6)** aus Ringsegmenten besteht, die in Summe die umlaufende Grenzfläche ganz oder teilweise überdecken.

6. Weitwinkeloptik nach einem der Ansprüche 1 bis 5, **dadurch gekennzeichnet, dass** die Auskopplungsstruktur **(6)** durch mindestens ein Ringprisma und/oder Ringprismensegmente gebildet wird.

7. Weitwinkeloptik nach einem der Ansprüche 1 bis 6, **dadurch gekennzeichnet, dass** die proximal zum Augeninneren angeordnete Linse sowie die Auskopplungsstruktur **(6)** einen voneinander abweichenden Brechungsindex aufweisen.

8. Weitwinkeloptik nach einem der Ansprüche 1 bis 7, **dadurch gekennzeichnet, dass** die Auskopplungsstruktur **(6)** ganz oder teilweise formschlüssig an die umlaufende Grenzfläche anschließt.

9. Weitwinkeloptik nach einem der Ansprüche 1 bis 7, **dadurch gekennzeichnet, dass** die Auskopplungsstruktur **(6)** ganz oder teilweise berührungslos und/oder relativbeweglich an die umlaufende Grenzfläche anschließt.

## Claims

1. Wide-angle optical unit for ophthalmological implants in an eye, comprising a lens system (2) arranged rotationally symmetrically about an optical axis (5), comprising at least two lenses (8-12) lying areally one on another and composed of materials having different optical refractive indexes, and a supplementary lens (4), which is arranged proximally with respect to the inside of the eye, **characterized in that** an optical coupling-out structure (6) is placed around the supplementary lens (4).

2. Wide-angle optical unit according to claim 1, **characterised in that** an additional mechanically displaceable lens combination (3) and/or lens body of adjustable focal length is provided.

3. Wide-angle optical unit according to claim 2, **characterised in that** the supplementary lens (4) arranged proximally with respect to the inside of the eye and/or the displaceable lens combination (3) and/or lens body of adjustable focal length comprises an annular circumferential limit surface, wherein the optical coupling-out structure (6) is placed on this.

4. Wide-angle optical unit according to any one of claims 1 to 3, **characterised in that** the coupling-out structure (6) comprises a non-constant structure cross- , section in the circumferential direction.

5. Wide-angle optical unit according to any one of claims 1 to 4, **characterised in that** the coupling-out structure (6) consists of ring segments, which in total cover the circumferential limit surface in whole or in part.

6. Wide-angle optical unit according to any one of claims 1 to 5, **characterised in that** the coupling-out structure (6) is formed by at least one ring prism and/or ring prism segments.

7. Wide-angle optical unit according to any one of claims 1 to 6, **characterised in that** the lenses arranged proximally with respect to the inside of the eye and the coupling-out structure exhibit refractive indices which differ from one another.

8. Wide-angle optical unit according to any one of claims 1 to 7, **characterised in that** the coupling-out structure (6) adjoins the circumferential limit surface in whole or in part, in positive fit.

9. Wide-angle optical unit according to one of claims 1 to 7, **characterised in that** the coupling-out structure (6) adjoins the circumferential limit surface, in whole or in part, free of contact and/or with relative movement.

## Revendications

1. Optique grand angle pour permettre la mise en place d'implants ophtalmologiques dans des yeux, comprenant un système de lentilles (2) montées de façon à présenter une symétrie de rotation autour d'un axe optique (5) et comprenant au moins deux lentilles (8, 12) positionnées à plat l'une sur l'autre en des matériaux ayant des indices de rétraction différents, ainsi qu'une lentille annexe (4) qui est montée dans une position proximale par rapport à la partie interne de l'oeil, **caractérisé en ce qu'**
une structure de sortie optique (6) est montée autour de la lentille annexe (4).

2. Optique grand angle selon la revendication 1,
**caractérisé en ce qu'**
il est en outre prévu une combinaison de lentilles (3) susceptible de coulisser mécaniquement et/ou un corps de lentilles ayant une focale variable.

3. Optique grand angle conforme à la revendication 2,
**caractérisé en ce que**
la lentille annexe (4) montée dans une position proximale par rapport à la partie interne de l'oeil et/ou la combinaison de lentilles susceptible de coulisser (3) et/ou le corps de lentilles de focale réglable comporte une surface limite périphérique annulaire, la structure de sortie optique (6) étant montée sur celle-ci.

4. Optique grand angle conforme à l'une des revendications 1 à 3,
**caractérisé en ce que**
la structure de sortie (6) a une section de structure non constante en direction périphérique.

5. Optique grand angle conforme à l'une des revendications 1 à 4,
**caractérisé en ce que**
la structure de sortie (6) est constituée par des segments annulaires qui recouvrent globalement la surface limite périphérique, partiellement ou en totalité.

6. Optique grand angle conforme à l'une des revendications 1 à 5,
**caractérisé en ce que**
la structure de sortie (6) est formée par au moins un prisme annulaire et/ou des segments de prisme annulaires.

7. Optique grand angle conforme à l'une des revendications 1 à 6,
**caractérisé en ce que**
la lentille montée dans une position proximale par rapport à la partie interne de l'oeil ainsi que la structure de sortie (6) ont des indices de réfraction différents.

8. Optique grand angle conforme à l'une des revendications 1 à 7,
**caractérisé en ce que**
la structure de sortie (6) se connecte totalement ou partiellement par une liaison par la forme sur la surface limite périphérique.

9. Optique grand angle conforme à l'une des revendications 1 à 7,
**caractérisé en ce que**
la structure de sortie (6) se connecte en totalité ou partiellement sans contact et/ou avec déplacement relatif sur la surface limite périphérique.
